# EUROPEAN PATENT APPLICATION

(11) **EP 4 534 109 A1**
(43) Date of publication of application: **09.04.2025**
(21) Application number: 23811664.4
(22) Date of filing: 15.05.2023
(51) Int. Cl.: A61K 49/00, C07D 407/04, C07F 7/10, C12Q 1/34, G01N 21/64

(54) **FLUORESCENT-PROBE SOLUTION**

(30) Priority: 27.05.2022 JP 2022086480
(71) Applicant: Goryo Chemical, Inc., Sapporo-shi, Hokkaido 060-0008 (JP)
(72) Inventor: FUJII, Taiga, Sapporo-shi, Hokkaido 060-0008 (JP); SUZUKI, Yuki, Sapporo-shi, Hokkaido 060-0008 (JP)
(74) Representative: Müller-Boré & Partner Patentanwälte PartG mbB
(86) International application number: PCT/JP2023/018050
(87) International publication number: WO 2023/228790

(57) **Abstract**

The present invention provides a fluorescent-probe solution in which a reduction in purity of a fluorescent probe is suppressed. The fluorescent-probe solution of the present invention includes: a compound represented by the formula (I) or a salt thereof; at least one kind of organic solvent selected from the group consisting of: dimethyl sulfoxide; an alcohol having 1 to 3 carbon atoms; and N,N-dimethylformamide; and water. A content of the organic solvent is from 5 vol% to 95 vol%, and a content of the water is from 5 vol% to 95 vol%.

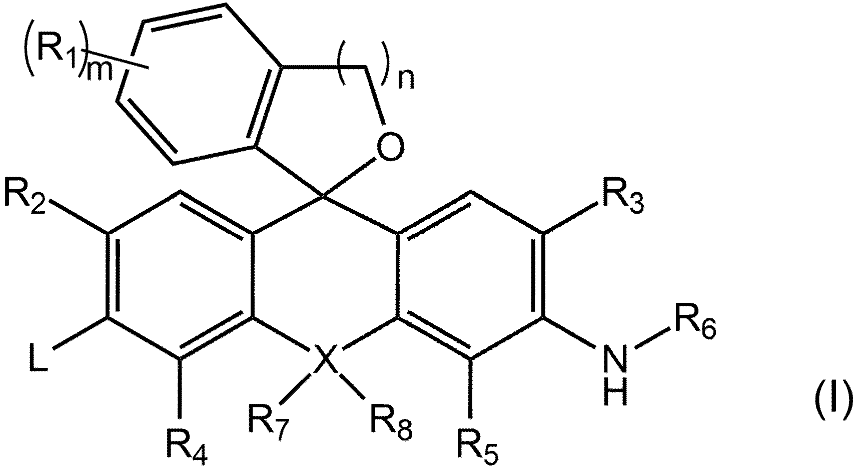

## Description

### Technical Field

The present invention relates to a fluorescent-probe solution.

### Background Art

In recent years, a fluorescent probe that can specifically detect cancer by evaluating the enzymatic activity of a sample has been progressively developed. For example, in Patent Literature 1, there is a disclosure of a fluorescent probe that can detect malignant and benign tumors of the mammary gland, lung adenocarcinoma, or lung squamous cell carcinoma by focusing on glycosidase activity as the enzymatic activity of a subject to be evaluated.

### Citation List

### Patent Literature

[PTL 1] WO 2020/175688 A1

### Summary of Invention

### Technical Problem

The inventors of the present invention have made investigations on practical use of the fluorescent probe of Patent Literature 1, but have found that the purity of the fluorescent probe may be reduced during storage.

The present invention has been made to solve the above-mentioned problem, and a primary object of the present invention is to provide a fluorescent-probe solution in which a reduction in purity of the fluorescent probe is suppressed.

### Solution to Problem

According to one aspect of the present invention, there is provided a fluorescent-probe solution, including: a compound represented by the following formula (I) or a salt thereof; at least one kind of organic solvent selected from the group consisting of dimethyl sulfoxide, an alcohol having 1 to 3 carbon atoms, and N,N-dimethylformamide; and water; wherein a content of the organic solvent is from 5 vol% to 95 vol%, and wherein a content of the water is from 5 vol% to 95 vol%: 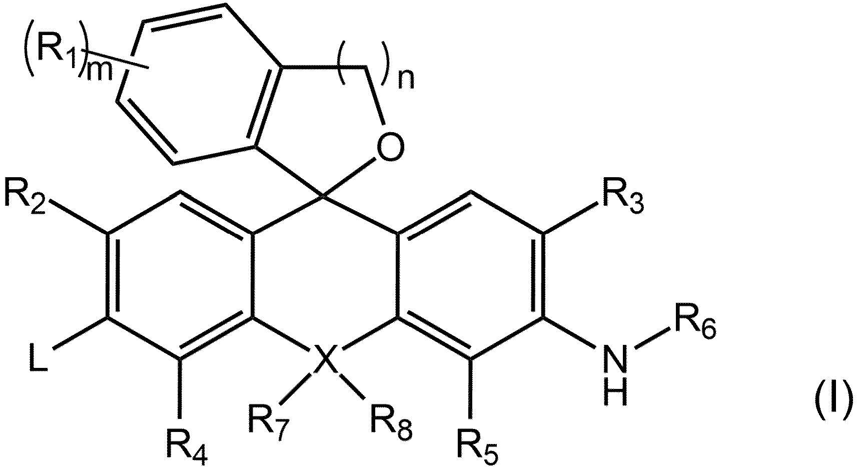 where:
R₁, if present, represents an identical or different monovalent substituent present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or a fluorinated alkyl group having 1 to 5 carbon atoms;
R₇ and R₈, if present, each independently represent an alkyl group having 1 to 6 carbon atoms or an aryl group;
X represents an oxygen atom, a silicon atom, or a carbon atom;
when X represents an oxygen atom, R₇ and R₈ are absent;
"n" represents an integer of from 1 to 3; and
L is selected from any groups of the following formulae (1) to (6).

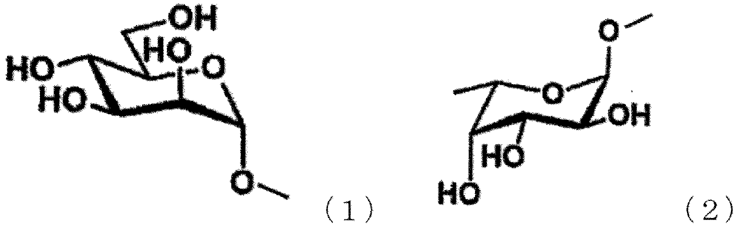

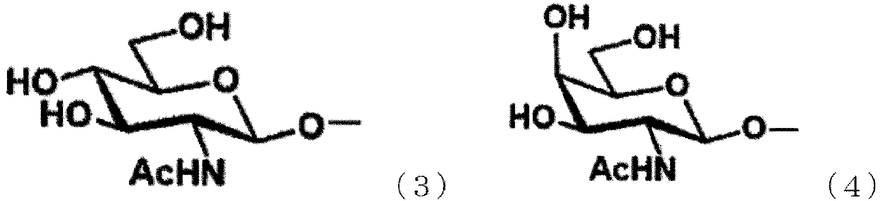

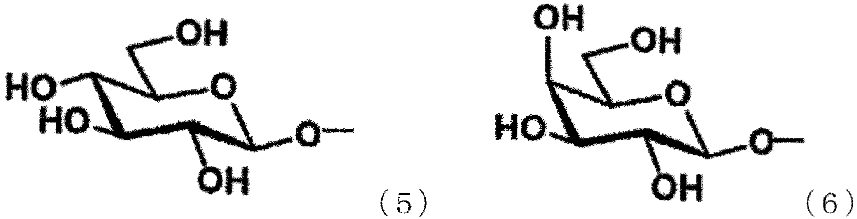

### Advantageous Effects of Invention

According to an embodiment of the present invention, there can be obtained a fluorescent-probe solution that can be stored over a long period of time while the reduction in purity of the compound represented by the formula (I) is suppressed by the presence of water in a solvent.

### Brief Description of Drawings

FIG. **1** is a graph showing the reduction amount of a purity (converted value) in storage of each of fluorescent-probe solutions of Test Examples 1 to 11 at room temperature for 3 years.
FIG. **2** is a graph showing the reduction amount of a purity (converted value) in storage of each of the fluorescent-probe solutions of Test Examples 1 to 11 at 50°C for 1 year.
FIG. **3** is a graph showing the increase amount of an oxidized form (converted value) in storage of each of the fluorescent-probe solutions of Test Examples 1 to 11 at 50°C for 1 year.
FIG. **4** is a graph showing the reduction amount of a purity (converted value) in storage of each of fluorescent-probe solutions of Test Examples 13 to 15 at room temperature for 3 years.
FIG. **5** is a graph showing the increase amount of an oxidized form (converted value) in storage of each of the fluorescent-probe solutions of Test Examples 13 to 15 at room temperature for 3 years.
FIG. **6** is fluorescence images of mammary gland clinical samples obtained through use of fluorescent-probe solutions for detection of Test Examples 12, 16, and 17.

### Description of Embodiments

Preferred embodiments of the present invention are described below. However, the present invention is not limited to these embodiments. Herein, the expression "from ··· to ···" representing a numerical range includes the upper limit and lower limit numerical values thereof. The embodiments may be combined with each other unless otherwise specified.

Herein, the "alkyl group" or the alkyl moiety of a substituent having an alkyl moiety (e.g., an alkoxy group) means an alkyl group having a linear structure, a branched structure, a cyclic structure, or combination thereof and having, for example, about 1 to 6 carbon atoms, preferably about 1 to 4 carbon atoms, more preferably about 1 to 3 carbon atoms, unless otherwise stated. More specific examples of the alkyl group may include a methyl group, an ethyl group, a n-propyl group, an isopropyl group, a cyclopropyl group, a n-butyl group, a sec-butyl group, an isobutyl group, a tert-butyl group, a cyclopropylmethyl group, a n-pentyl group, and a n-hexyl group.

Herein, the "halogen atom" may refer to any of a fluorine atom, a chlorine atom, a bromine atom, or an iodine atom, and preferably refers to a fluorine atom, a chlorine atom, or a bromine atom.

### A. Fluorescent-probe Solution

A fluorescent-probe solution according to an embodiment of the present invention includes: a compound represented by the formula (I) (hereinafter sometimes referred to as "compound (I)") or a salt thereof; at least one kind of organic solvent selected from the group consisting of: dimethyl sulfoxide; an alcohol having 1 to 3 carbon atoms; and N,N-dimethylformamide; and water. The content of the organic solvent in the fluorescent-probe solution is from 5 vol% to 95 vol%, and the content of water is from 5 vol% to 95 vol%. The fluorescent-probe solution may further include any appropriate additive as required. Herein, the "compound represented by the formula (I) or a salt thereof" is sometimes collectively referred to as "compound (I)." Herein, the content (vol%) of each of the organic solvent, water, and an additive that is a liquid at 25°C in the fluorescent-probe solution is synonymous with the volume ratio (vol%) of each of the components before mixing with respect to the sum of the volumes of the components before mixing, and a value measured in an air atmosphere at 25°C and 1,013.25 hPa may be applied. The blending amounts of the compound represented by the formula (I) and the other additives are small, and hence their influences on the volume of the fluorescent-probe solution may be ignored. According to another aspect of the present invention, there can be provided a method of producing the fluorescent-probe solution, the method including: mixing the compound (I) or the salt thereof, at least one kind of organic solvent selected from the group consisting of: dimethyl sulfoxide; an alcohol having 1 to 3 carbon atoms; and N,N-dimethylformamide; water; and an additive serving as an optional component, wherein the ratio of the organic solvent with respect to the total volume of the organic solvent, the water, and the additive that is a liquid at 25°C before mixing is from 5 vol% to 95 vol%, and the mixing ratio of the water with respect thereto is from 5 vol% to 95 vol%.

### A-1. Compound represented by Formula (I)

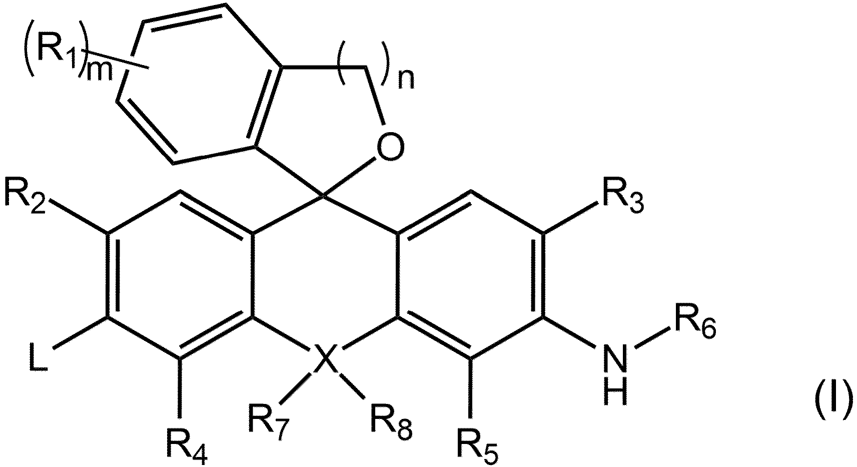

In the general formula (I), R₁, if present, represents an identical or different monovalent substituent present on a benzene ring. Examples of the monovalent substituent include a halogen and an alkyl group that may be substituted.

"m" represents an integer of from 0 to 4. In one embodiment, "m" represents 0, and thus R₁ is absent. That is, a benzene ring bonded to a xanthene skeleton is an unsubstituted benzene ring.

In the general formula (I), R₂ and R₃ each independently represent a hydrogen atom, an alkyl group having 1 to 6 carbon atoms, or a halogen atom.

When R₂ and R₃ each represent an alkyl group, the alkyl group may be substituted. Specifically, the alkyl group may have one or two or more halogen atoms, carboxy groups, sulfonyl groups, hydroxy groups, amino groups, alkoxy groups, or the like. For example, the alkyl group represented by R₂ or R₃ may be a halogenated alkyl group, a hydroxyalkyl group, or a carboxyalkyl group.

It is preferred that R₂ and R₃ each independently represent a hydrogen atom or a halogen atom. When R₂ and R₃ each represent a halogen atom, it is preferred that both R₂ and R₃ represent fluorine atoms or chlorine atoms. In one embodiment, both R₂ and R₃ represent hydrogen atoms.

R₄ and R₅ each independently represent a hydrogen atom, an alkyl group having 1 to 6 carbon atoms, or a halogen atom, and are the same as those described for R₂ and R₃. In one embodiment, both R₄ and R₅ represent hydrogen atoms.

R₆ represents a hydrogen atom, an alkyl group having 1 to 5 carbon atoms, or a fluorinated alkyl group having 1 to 5 carbon atoms. The alkyl group represented by R₆ is preferably a methyl group or an ethyl group. The fluorinated alkyl group represented by R₆ is preferably -CH₂-CF₃ or -CH₂-CH₂-CF₃. In one embodiment, R₆ represents -CH₂-CF₃.

In the general formula (I), R₇ and R₈, if present, each independently represent an alkyl group having 1 to 6 carbon atoms or an aryl group. It is preferred that R₇ and R₈ each independently represent an alkyl group having 1 to 3 carbon atoms, and it is more preferred that both R₇ and R₈ represent methyl groups.

The alkyl group represented by R₇ or R₈ may be substituted. Specifically, the alkyl group may have one or two or more halogen atoms, carboxy groups, sulfonyl groups, hydroxy groups, amino groups, alkoxy groups, or the like. For example, the alkyl group represented by R₇ or R₈ may be a halogenated alkyl group, a hydroxyalkyl group, or a carboxyalkyl group.

When R₇ or R₈ represents an aryl group, the aryl group may be any of a monocyclic aromatic group or a condensed aromatic group, and an aryl ring may include one or two or more ring-forming heteroatoms (e.g., nitrogen atoms, oxygen atoms, or sulfur atoms). The aryl group is preferably a phenyl group. One or two or more substituents may be present on the aryl ring. For example, one or two or more halogen atoms, carboxy groups, sulfonyl groups, hydroxy groups, amino groups, or alkoxy groups may be present as the substituents.

In addition, when X to be described later represents an oxygen atom, R₇ and R₈ are absent.

X represents an oxygen atom, a silicon atom, or a carbon atom. In one embodiment, X represents an oxygen atom.

"n" represents an integer of from 1 to 3, preferably 1.

In the general formula (I), L is selected from any groups of the following formulae (1) to (6).

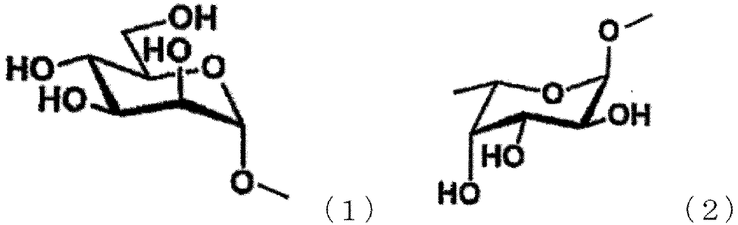

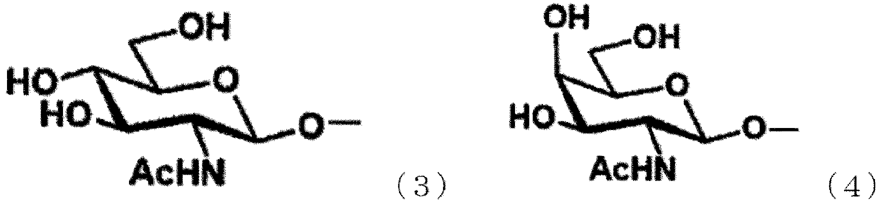

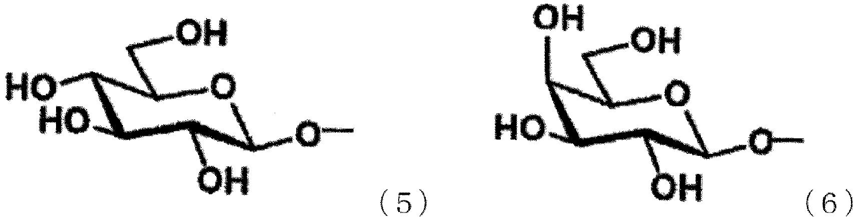

In one embodiment, the compound (I) is a compound represented by the following formula (Ia) where L is the same as L in the formula (I), and is selected from any groups of the formulae (1) to (6).

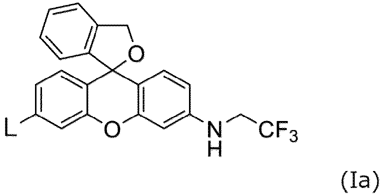

The compound (I) may have one or two or more asymmetric carbon atoms depending on the kinds of the substituents. In the present invention, for example, a stereoisomer, such as an optically active substance based on one or two or more asymmetric carbon atoms or a diastereomer based on two or more asymmetric carbon atoms, may be used, and any appropriate mixture of stereoisomers or a racemate may also be used.

The salt of the compound (I) may be an acid addition salt or a base addition salt. Examples of the acid addition salt may include: mineral acid salts, such as a hydrochloride, a sulfurate, and a nitrate; and organic acid salts, such as a methanesulfonate, a p-toluenesulfonate, an oxalate, a citrate, and a tartrate. Examples of the base addition salt may include: metal salts, such as a sodium salt, a potassium salt, a calcium salt, and a magnesium salt; and organic amine salts, such as an ammonium salt and a triethylamine salt. In addition to the foregoing, a salt with an amino acid such as glycine may be formed. The compound (I) or the salt thereof may be present as a hydrate or a solvate, and in the present invention, such substance may also be used.

The compound (I) and the salt thereof may be synthesized by any appropriate method. A method of synthesizing the compound (I) and the salt thereof is, for example, a synthesis method described in Patent Literature 1.

In one embodiment, the content of the compound (I) in the fluorescent-probe solution may be, for example, from 0.1 mM to 100 mM, and may also be, for example, from 0.5 mM to 100 mM, preferably from 1 mM to 60 mM, more preferably from 3 mM to 20 mM. The fluorescent-probe solution including the compound (I) at such high concentration may be diluted with a diluent, such as PBS or physiological saline, for use.

### A-2. Organic Solvent

The organic solvent is selected from the group consisting of: dimethyl sulfoxide (DMSO); an alcohol having 1 to 3 carbon atoms; and N,N-dimethylformamide (DMF). Preferred examples of the alcohol having 1 to 3 carbon atoms include ethanol and isopropanol. Each of those organic solvents has satisfactory miscibility with water and is capable of satisfactorily dissolving the compound (I). One kind of organic solvent selected from the above-mentioned group may be used alone, or two or more kinds thereof may be used in combination.

The content of the organic solvent in the fluorescent-probe solution is typically from 5 vol% to 95 vol%. When the content is less than 5 vol%, the solubility of the compound (I) may be reduced. In contrast, when the content is more than 95 vol%, an improving effect on the stability of the compound (I) may be reduced. The content may be preferably 10 vol% or more, more preferably 20 vol% or more, and may be set to, for example, 40 vol% or more. The content is preferably 90 vol% or less, more preferably 80 vol% or less, still more preferably 75 vol% or less.

In one embodiment, the content of the organic solvent in the fluorescent-probe solution may be, for example, 40 vol% or more, preferably 45 vol% or more, more preferably 50 vol% or more, for example, 55 vol% or more or 60 vol% or more, and may be preferably 90 vol% or less, more preferably 80 vol% or less, still more preferably 75 vol% or less. According to this embodiment, even when a solubilizer to be described later is not used, the improving effect on the stability of the compound (I) exhibited by addition of water can be suitably obtained while the solubility of the compound (I) is secured.

In one embodiment, the content of the organic solvent in the fluorescent-probe solution may be, for example, from 10 vol% to 90 vol%, preferably from 20 vol% to 80 vol%, more preferably from 25 vol% to 70 vol%, still more preferably from 30 vol% to 60 vol%, even still more preferably from 35 vol% to 45 vol%. When the content of the organic solvent is reduced, the improving effect on the stability of the compound (I) (e.g., a suppressing effect on a reduction in purity thereof) can be more suitably obtained. Even when the content of the organic solvent is relatively low, the use of the solubilizer to be described later can secure the solubility of the compound (I) and suitably achieve the improving effect on the stability of the compound (I) exhibited by addition of water.

In one embodiment, the organic solvent contains DMSO. In this embodiment, the usage amount of DMSO may be 50 vol% or more, preferably 75 vol% or more, for example, 100 vol%, with respect to the entire organic solvent.

### A-3. Water

When water is blended in the fluorescent-probe solution, the stability of the compound (I) in the fluorescent-probe solution can be improved. Purified water, such as RO water, ion-exchanged water, or distilled water, is preferably used as the water.

The content of water in the fluorescent-probe solution is typically from 5 vol% to 95 vol%. When the content is less than 5 vol%, the improving effect on the stability of the compound (I) may be reduced. Meanwhile, when the content is more than 95 vol%, the content of the organic solvent is reduced, and as result, the solubility of the compound (I) may be reduced. The content is preferably 10 vol% or more, more preferably 20 vol% or more, still more preferably 25 vol% or more. The content may be preferably 90 vol% or less, more preferably 80 vol% or less, and may be set to, for example, 60 vol% or less.

In one embodiment, the content of water in the fluorescent-probe solution may be, for example, 10 vol% or more, preferably 20 vol% or more, more preferably 25 vol% or more, and may be preferably 60 vol% or less, more preferably 55 vol% or less, still more preferably 50 vol% or less, for example, 45 vol% or less or 40 vol% or less. According to this embodiment, even when the solubilizer to be described later is not used, the improving effect on the stability of the compound (I) can be obtained while the solubility of the compound (I) is secured.

In one embodiment, the content of water in the fluorescent-probe solution may be, for example, from 10 vol% to 90 vol%, preferably from 20 vol% to 80 vol%, more preferably from 30 vol% to 75 vol%, still more preferably from 40 vol% to 70 vol%, even still more preferably from 55 vol% to 65 vol%. When the content of water is increased, the improving effect on the stability of the compound (I) (e.g., a suppressing effect on a reduction in purity thereof) can be more suitably obtained. Even when the content of the organic solvent is relatively low, the use of the solubilizer to be described later can secure the solubility of the compound (I) and suitably achieve the improving effect on the stability of the compound (I) exhibited by addition of water.

### A-4. Additive

The additive is an optional component used as required. Accordingly, the fluorescent-probe solution may include only the three components described above, that is, the compound (I), the organic solvent, and water, or may include the additive in addition to these components. The total content of the organic solvent and water in the fluorescent-probe solution according to the embodiment of the present invention may be, for example, from 95 vol% to 100 vol% (e.g., from 95 vol% to 99.9 vol%), preferably from 99 vol% to 100 vol% (e.g., from 99 vol% to 99.8 vol%).

The additive may be appropriately selected in accordance with purposes. Specific examples of the additive include a buffer, a tonicity agent, and a solubilizer. Examples of the buffer include a phosphate buffer, a Tris buffer, a citrate buffer, and a borate buffer. Examples of the tonicity agent include sodium chloride and potassium chloride. Examples of the solubilizer include surfactants, such as a cationic surfactant, a nonionic surfactant, an amphoteric surfactant, and a nonionic surfactant. Of those, a nonionic surfactant is preferred. Examples of the nonionic surfactant include polyethylene glycol, glycerin fatty acid esters, polysorbates (e.g., Tween^{™} series), sorbitan fatty acid esters (e.g., RHEODOL^{™} series), polyoxyethylene alkyl ethers (e.g., EMULGEN^{™} series), polyoxyethylene alkyl phenyl ethers (e.g., Triton-X^{™} series), polyoxyethylene aryl ethers, polyoxyalkylene derivatives, alkyl alkanolamides, polyoxyethylene alkylamines, alkyl imidazolines, polyoxyethylene hydrogenated castor oils, aliphatic alcohol ethoxylates, and polyoxyethylene polyoxypropylene glycols (e.g., Poloxamer). The additives may be used alone or in combination thereof.

The blending amount of the additive may be appropriately selected by a person skilled in the art. For example, the content of the buffer in the fluorescent-probe solution may be, for example, from 1 mM to 100 mM, preferably from 5 mM to 20 mM. The content of the solubilizer in the fluorescent-probe solution may be, for example, from 0.05 w/v% to 5.0 w/v%, preferably from 0.5 w/v% to 3.0 w/v%. When the additive is a liquid (e.g., a liquid at 25°C), the content of the additive may be, for example, from 0.01 vol% to 5.0 vol%, preferably 0.02 vol% to 1.0 vol%.

### A-5. Preparation Method

The fluorescent-probe solution may be prepared by mixing the respective components at the above-mentioned contents (volume ratios before mixing) to dissolve the compound (I). In one embodiment, the fluorescent-probe solution may be prepared by dissolving the compound (I) in a mixed solution containing the organic solvent and water. In another embodiment, the fluorescent-probe solution may be prepared by dissolving the compound (I) in the organic solvent and mixing the obtained solution with water and the optional additive. The pH of the fluorescent-probe solution is, for example, from 5 to 9, preferably from 6 to 8, more preferably from 6.5 to 7.8.

The fluorescent-probe solution may be used as it is, or may be diluted with water, a buffer solution, physiological saline, or the like for use. The concentration of the fluorescent-probe solution at the time of use may be, for example, from 5 µM to 200 µM, preferably from 10 µM to 100 µM, more preferably from 40 µM to 60 µM in terms of concentration of the compound (I). The buffer solution to be used for the dilution may be appropriately selected by a person skilled in the art. For example, PBS or a Tris hydrochloride buffer solution may be used. The concentration of the buffer solution may be, for example, from 1 mM to 100 mM, preferably from 5 mM to 20 mM. The pH of the buffer solution may be, for example, from 6 to 8, more preferably from 7.2 to 7.6, still more preferably 7.4.

In one embodiment, the fluorescent-probe solution may be produced as a vial preparation and stored in a vial having an opening sealed with a rubber stopper. Specifically, a vial preparation may be produced by filling the vial with the fluorescent-probe solution, plugging the opening of the vial with a rubber stopper, and screwing a cap made of aluminum or the like. When the fluorescent-probe solution including the compound (I) is produced as a vial preparation, there may occur a problem in that the purity is reduced in a storage period. However, according to the fluorescent-probe solution of the embodiment of the present invention, both the solubility and stability of the compound (I) can be achieved by the presence of water in the organic solvent.

A glass material such as borosilicate glass or a resin material such as polypropylene may be used as a material for forming the vial. Of those, a glass material is preferred. The vial may be colorless or colored.

A material for forming the rubber stopper is, for example, a butyl rubber (IIR), an isoprene rubber (IR), a butadiene rubber (BR), a styrene-butadiene rubber (SBR), a fluorine rubber (FKM), or an ethylene propylene rubber (EPDM). When a rubber stopper made of a butyl rubber is used, the effects of the present invention can be more suitably obtained. The rubber stopper may be laminated. When a laminated rubber stopper is used, the effects of the present invention can also be suitably obtained.

The purity of the compound (I) in the fluorescent-probe solution immediately after preparation (e.g., within 12 hours after preparation) may be, for example, 95% or more, preferably 98% or more, more preferably 99% or more. The purity of the compound (I) in the fluorescent-probe solution may be measured, for example, by a method described in Examples.

The reduction amount of the purity of the compound (I) when the fluorescent-probe solution is stored at room temperature (typically, 25°C) for 3 years (the purity immediately after formulation - the purity after storage for 3 years) is, for example, 3.0% or less, preferably 2.0% or less, more preferably 1.5% or less, still more preferably 1.0% or less. The reduction amount of the purity of the compound (I) when the fluorescent-probe solution is stored at room temperature for 3 years may be a value calculated by converting the reduction amount of the purity when the fluorescent-probe solution is stored at room temperature for about 1 month into that for 3 years.

In one embodiment, the reduction amount of the purity of the compound (I) when the fluorescent-probe solution is stored at 50°C for 1 year (the purity immediately after formulation - the purity after storage for 1 year) is, for example, 7.0% or less, preferably 5.0% or less, more preferably 4.0% or less, still more preferably 3.0% or less. The reduction amount of the purity of the compound (I) when the fluorescent-probe solution is stored at 50°C for 1 year may be a value calculated by converting the reduction amount of the purity when the fluorescent-probe solution is stored at 50°C for about 1 month to that for 1 year.

### B. Application of Fluorescent-Probe Solution

The compound (I) is a glycosidase-reactive fluorescent probe. Specifically, the compound (I) having the group of the formula (1) as L in the formula (I) is an α-mannosidase-reactive fluorescent probe, the compound (I) having the group of the formula (2) is an α-L-fucosidase-reactive fluorescent probe, the compound (I) having the group of the formula (3) is a β-hexosaminidase-reactive fluorescent probe, the compound (I) having the group of the formula (4) is a β-N-acetylgalactosaminidase-reactive fluorescent probe, the compound (I) having the group of the formula (5) is a β-glucosidase, and the compound (I) having the group of the formula (6) is a β-galactosidase-reactive fluorescent probe.

The use of the compound (I) enables visualization and evaluation of the glycosidase activity of a surgically-resected tissue sample without homogenization.

In one embodiment, the compound (I) having any one of the groups of the formulae (1) to (6) as L in the formula (I) is capable of detecting both breast cancer and a benign tumor. Accordingly, the fluorescent-probe solution including such compound (I) is effective for diagnostic imaging of breast cancer and a benign tumor of the mammary gland, for example, mammary fibroadenoma and invasive mammary ductal carcinoma, and noninvasive mammary ductal carcinoma.

The fluorescent-probe solution described in the section A may be used in combination with a γ-glutamyl transpeptidase (GGT) activity-detecting probe. For example, when a red GGT activity-detecting probe and the compound (I) are used in combination for detection of breast cancer, the GGT activity-detecting probe emits red fluorescence equally in both breast cancer (malignant tumor) and a benign tumor, and the compound (I) emits strong green fluorescence, in particular, in the benign tumor. Accordingly, a combination of images of both the red fluorescence and the green fluorescence allows for formation of an image in which a breast cancer tissue is represented by red and a benign tumor tissue is represented by yellow. Thus, the use of the compound (I) in combination with the red GGT activity-detecting probe makes it possible to determine that a portion colored by fluorescence emitted by both the compound (I) and the GGT activity-detecting probe is a benign tumor portion and that a portion colored by fluorescence emitted by only the GGT activity-detecting probe is a breast cancer (malignant tumor) portion, and to discriminate between a benign tumor and a malignant tumor. Accordingly, the efficiency of partial resection can be improved without excision of the benign tumor portion in a surgical operation.

The above-mentioned GGT activity-detecting probe is, for example, a compound represented by the general formula (II) or a salt thereof, which is described in Patent Literature 1.

Breast cancer and a benign tumor of the mammary gland may be detected by a method including: (Step a1) applying the fluorescent-probe solution including the compound (I) having any one of the groups of the formulae (1) to (6) as L to a clinical sample of the breast; and (Step b1) measuring a fluorescent image of the clinical sample of the breast to which the fluorescent-probe solution has been applied.

(Step a1) The applying the fluorescent-probe solution to a clinical sample of the breast may be performed, for example, by locally spraying the fluorescent-probe solution on the clinical sample of the breast or immersing the clinical sample of the breast (excised sample) in the fluorescent-probe solution. (Step b1) The measuring a fluorescent image may be performed by obtaining the fluorescent image of the clinical sample to which the fluorescent-probe solution has been applied through use of a fluorescent observation device.

In one embodiment, the compound (I) having the group of the formula (5) or (6) as L in the formula (I) is effective for specific imaging of main lung cancer including lung adenocarcinoma in addition to lung squamous cell carcinoma that cannot be detected through use of an aminopeptidase-reactive fluorescent probe. Accordingly, the fluorescent-probe solution including such compound (I) can specifically detect lung adenocarcinoma or lung squamous cell carcinoma.

Lung adenocarcinoma or lung squamous cell carcinoma may be detected by a method including: (Step a2) applying the fluorescent-probe solution including the compound (I) having the group of the formula (5) or (6) as L to a clinical sample of lung adenocarcinoma or lung squamous cell carcinoma; and (Step b2) measuring a fluorescent image of the clinical sample of lung adenocarcinoma or lung squamous cell carcinoma to which the fluorescent-probe solution has been applied.

(Step a2) The applying the fluorescent-probe solution to a clinical sample of lung adenocarcinoma or lung squamous cell carcinoma may be performed, for example, by locally spraying the fluorescent-probe solution on the clinical sample of lung adenocarcinoma or lung squamous cell carcinoma or immersing the excised sample in the fluorescent-probe solution. (Step b2) The measuring a fluorescent image may be performed by obtaining the fluorescent image of the clinical sample to which the fluorescent-probe solution has been applied through use of a fluorescent observation device.

### Examples

The present invention is specifically described below by way of Examples. However, the present invention is by no means limited to these Examples.

### [Synthesis Example 1] Synthesis of HMRef-αMan

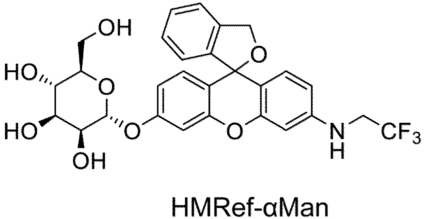

Dichloromethane (7.2 mL) was added to HMRef (109 mg, 0.273 mmol), penta-O-acetyl-D-mannopyranoside (1.07 g, 2.73 mmol), and MS4A (200 mg), and the mixture was stirred at room temperature for 1 hour. To the reaction solution, boron trifluoride diethyl ether complex (2.4 mL, 19.1 mmol) was added, and the mixture was stirred at room temperature for 16 hours. After the mixture had been cooled to 0°C, a saturated sodium hydrogen carbonate aqueous solution was added thereto, and the reaction solution was filtered through Celite. The filtrate was extracted with dichloromethane, the organic phase was washed with saturated saline and dried over sodium sulfate, and the solvent was then evaporated under reduced pressure. The residue was dissolved in methanol (4 mL), and a 28% sodium methoxide methanol solution (1 mL) was added thereto, and the mixture was stirred at room temperature for 1.5 hours. The mixture was neutralized with a 10% phosphoric acid aqueous solution and extracted with dichloromethane, the organic phase was washed with saturated saline and dried over sodium sulfate, and the solvent was then evaporated under reduced pressure. The residue was purified by column chromatography to provide a target product (18.5 mg, 12%).

### [Test Examples 1 to 12]

Components were mixed at each composition shown in Table 1 to provide each fluorescent-probe solution. Specifically, HMRef-αMan obtained in Synthesis Example 1 was dissolved in DMSO at a concentration of 10 mM, and 125 µL of the resultant solution and a required amount of water or a phosphate buffer solution (pH: 7.4) were mixed to provide the fluorescent-probe solution.

**Table 1**

| Test Example No. | 1 | 2 | 3 | 4 | 5 | 6 | 7 | 8 | 9 | 10 | 11 | 12 |
|---|---|---|---|---|---|---|---|---|---|---|---|---|
| DMSO (v/v%) | 100 | 90 | 80 | 70 | 60 | 50 | 40 | 60 | 60 | 60 | 60 | 50 |
| Water (v/v%) | 0 | 10 | 20 | 30 | 40 | 50 | 60 | 40 | 40 | 40 | 40 | 50 |
| Phosphate (mM) | 0 | 0 | 0 | 0 | 0 | 0 | 0 | 10 | 5 | 2.5 | 1 | 10 |

A 25 mL glass vial (manufactured by Daiwa Special Glass Co., Ltd.) was filled with 0.125 mL to 0.312 mL of the fluorescent-probe solution obtained in each of Test Examples 1 to 11, and the opening of the glass vial was plugged with a rubber stopper (No. 20-T manufactured by Maruemu Corporation), and then tightly sealed with an aluminum cap over the rubber stopper. The vial filled with each of the fluorescent-probe solutions was stored at 25°C or 50°C in the dark for from 25 days to 32 days.

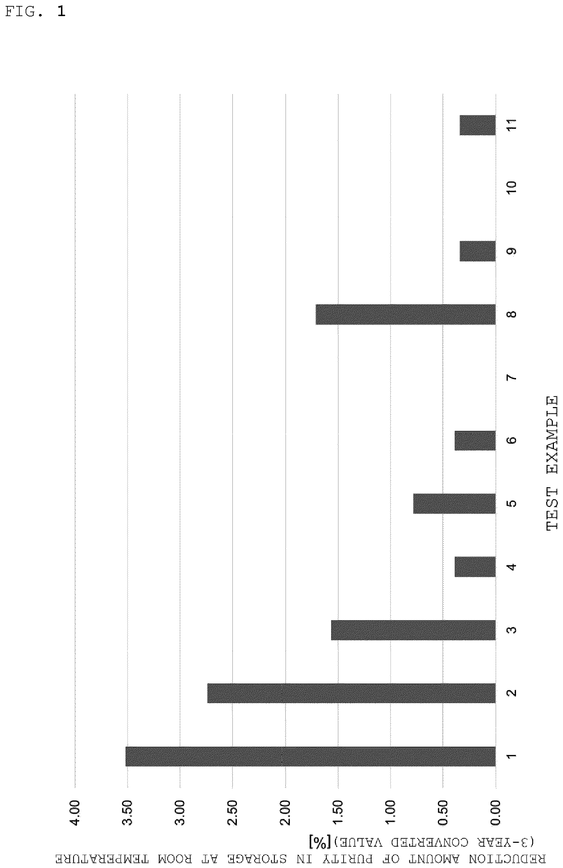

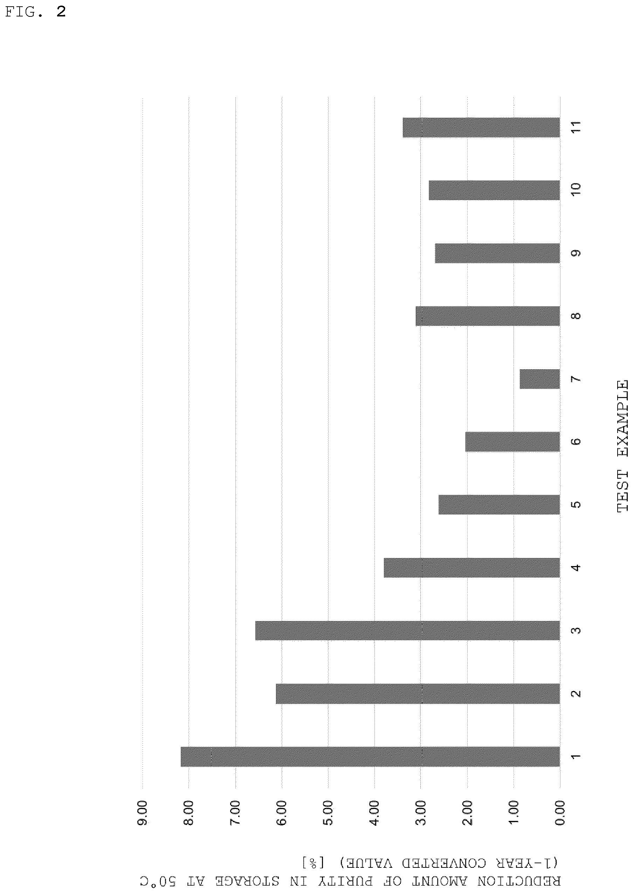

The purity of HMRef-αMan in each of the fluorescent-probe solutions within 12 hours after preparation, and that in each of the fluorescent-probe solutions after storage for from 25 days to 32 days in the vials were measured as described below, and the difference between the purities (the former - the latter) was divided by the number of storage days. Thus, the reduction amount of the purity per day was calculated. In addition, the reduction amount of the purity (converted value) in storage (3 years) at room temperature or in storage (1 year) at 50°C was calculated from the reduction amount of the purity per day. The results are shown in FIG. **1** and FIG. **2****.** All the purities of HMRef-αMan within 12 hours after the preparation of the fluorescent-probe solutions obtained in Test Examples 1 to 11 were 99% or more.

### <Method of measuring HMRef-αMan Solution>

HPLC analysis was performed under the following conditions through use of a liquid chromatography system (UltiMate 3000, Thermo Fisher Scientific Inc.), and the ratio of the peak area of HMRef-αMan with respect to the entire peak area was calculated as a purity.
Column: Meteoric core C8, 5 µm, 4.6×150 mm (YMC Co., Ltd.)
Column temperature: 25°C
Mobile phase A: water/TFA (1,000:1)
Mobile phase B: acetonitrile/TFA (1,000:1)
Liquid-feeding amount: 1.0 mL/min.
Wavelength: 254 nm
Liquid feeding to mobile phase: The liquid was fed under the conditions shown in the following table.

**Table 2**

| Time after injection (min) | Mobile phase A (vol%) | Mobile phase B (vol%) |
|---|---|---|
| 0 to 20 | 84 to 59 | 16 to 41 |

For the fluorescent-probe solutions after storage for from 25 days to 32 days in the vials, a peak resolved under the same conditions as those of the above-mentioned liquid chromatography was subjected to mass spectrometry analysis, and as a result, an analog (oxidized form) in which two hydrogen atoms were lost from HMRef-αMan was observed as a main analog.

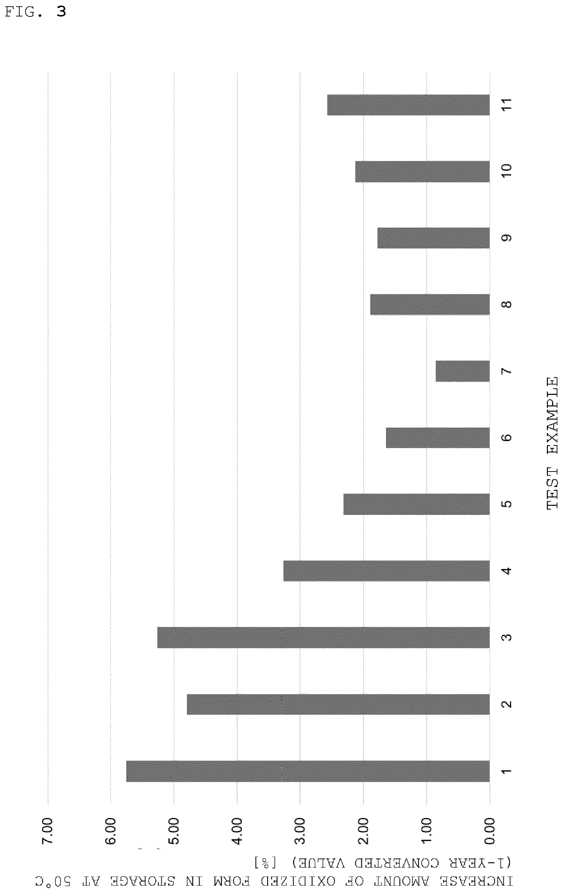

The content (the ratio of the oxidized form peak area with respect to the entire peak area: %) of the oxidized form of HMRef-αMan in each of the fluorescent-probe solutions within 12 hours after preparation, and that in each of the fluorescent-probe solutions after storage at 50°C for from 25 days to 32 days in the vials were measured in the same manner as described above, and the difference between the contents (the latter - the former) was divided by the number of storage days. Thus, the increase amount of the oxidized form per day was calculated. Further, a value obtained by converting the increase amount of the oxidized form per day into that for 1 year was calculated as the increase amount of the oxidized form in storage for 1 year. The results are shown in FIG. **3****.**

As shown in FIG. **1** and FIG. **2****,** a reduction in purity of HMRef-αMan was suppressed by the presence of water in the solvent, and stability during storage was improved. The effects were similarly recognized even in the presence of a phosphate. In addition, from FIG. **2** and FIG. **3****,** it is found that the production of the oxidized form of HMRef-αMan leads to the reduction in purity. The oxidation of HMRef-αMan is expected to occur in a fluorescence skeleton of the formula (I), and hence the effects of the present invention are inferred to be obtained regardless of the kinds of sugars in the residue L.

### [Test Examples 13 to 15]

Components were mixed at each composition shown in Table 3 to provide each fluorescent-probe solution. Specifically, HMRef-αMan obtained in Synthesis Example 1 was dissolved in DMSO at a concentration of 10 mM, and 100 µL of the resultant DMSO solution, 100 µL of a Tween 80 aqueous solution containing Tween 80 (manufactured by Tokyo Chemical Industry Co., Ltd.) at a concentration of 2 w/v%, and a required amount of water were mixed to provide the fluorescent-probe solution. The addition of Tween 80 as a solubilizer made it possible to dissolve the fluorescent probe even when the blending amount of the organic solvent was small.

**Table 3**

| Text Example No. | 13 | 14 | 15 |
|---|---|---|---|
| DMSO (v/v%) | 50 | 25 | 10 |
| Tween 80 aqueous solution (v/v%) | 50 | 25 | 10 |
| Water (v/v%) | 0 | 50 | 80 |

A glass vial was filled with the fluorescent-probe solution obtained in each of Test Examples 13 to 15, and sealed with a rubber stopper. The vial filled with each of the fluorescent-probe solutions was stored at 25°C in the dark for 85 days, and analysis was performed by liquid chromatography.

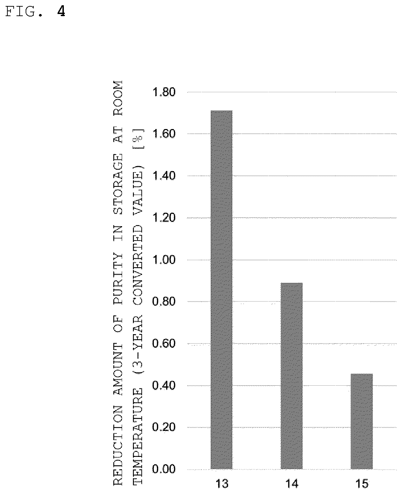

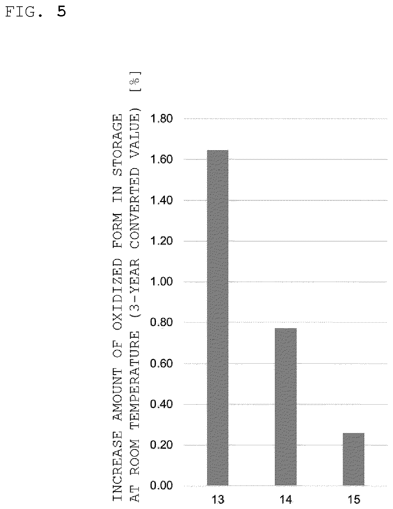

In the same manner as in Test Examples 1 to 11, the purity of HMRef-αMan and the content of the oxidized form thereof in each of the fluorescent-probe solutions within 12 hours after preparation, and those in each of the fluorescent-probe solutions after storage for 85 days in the vials were measured, and the reduction amount of the purity and the increase amount of the oxidized form in 3 years were calculated as converted values. The results are shown in FIG. **4** and FIG. **5****.** All the purities of HMRef-αMan within 12 hours after the preparation of the fluorescent-probe solutions obtained in Test Examples 13 to 15 were 99% or more.

As shown in FIG. **4** and FIG. **5****,** it is found that a reduction in purity of HMRef-αMan is suppressed even when the content of the organic solvent is reduced by adding the solubilizer, and the production of the oxidized form of HMRef-αMan leads to the reduction in purity. The obtained results reveal that a higher content of water is more effective for stabilization, and a probe solution having a higher content of water can be prepared by adding the solubilizer.

### [Test Example 16]

HMRef-αMan obtained in Synthesis Example 1 was dissolved at a concentration of 1.67 mM in an aqueous solution containing 6.5 w/v% mannitol, a 10 mM phosphate, and 0.33 w/v% Tween 80 (manufactured by Tokyo Chemical Industry Co., Ltd.) to provide a fluorescent-probe solution. The obtained fluorescent-probe solution was freeze-dried with a freeze-dryer (FDU-1200, TOKYO RIKAKIKAI CO., LTD.) to provide white powder.

### [Test Example 17]

HMRef-αMan obtained in Synthesis Example 1 was dissolved at a concentration of 1.67 mM in an aqueous solution containing 6.5 w/v% mannitol, a 10 mM phosphate, and 1.67 w/v% polyoxyethylene polyoxypropylene glycol (manufactured by FUJIFILM Wako Pure Chemical Corporation) to provide a fluorescent-probe solution. The obtained fluorescent-probe solution was freeze-dried with a freeze-dryer (FDU-1200, TOKYO RIKAKIKAI CO., LTD.) to provide white powder.

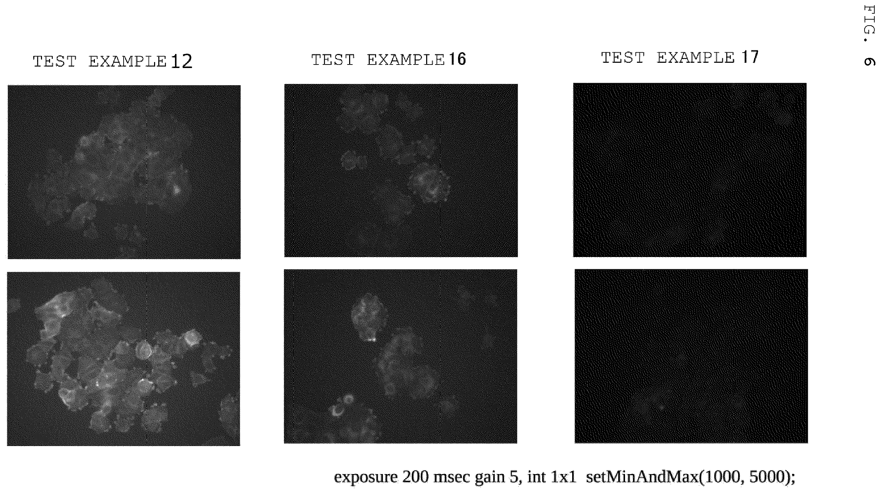

MCF-7 cells were plated in a glass bottom dish and cultured for from 1 day to 2 days (D-MEM high glucose + 10% FBS). The culture solution was removed, a fluorescent-probe solution for detection prepared by diluting or dissolving the fluorescent-probe solution of Test Example 12 or the white powder obtained in each of Test Examples 16 and 17 with PBS so that the concentration of HMRef-αMan was 10 µM was added thereto, and after 20 minutes, the cells were photographed with a fluorescent microscope (Leica DMI 6000 CS). The results are shown in FIG. **6****.**

As shown in FIG. **6****,** it is found that the fluorescent-probe solutions obtained by freeze-drying followed by dissolution each have a fluorescence intensity lower than that of the fluorescent-probe solution of Test Example 12, and have a lower detection capability.

### Industrial Applicability

The fluorescent-probe solution according to the embodiment of the present invention can be suitably used in detection of malignant and benign tumors of the mammary gland, lung adenocarcinoma, lung squamous cell carcinoma, or the like.

## Claims

1. A fluorescent-probe solution, comprising:
a compound represented by the following formula (I) or a salt thereof;
at least one kind of organic solvent selected from the group consisting of: dimethyl sulfoxide; an alcohol having 1 to 3 carbon atoms; and N,N-dimethylformamide; and
water,
wherein a content of the organic solvent is from 5 vol% to 95 vol%, and
wherein a content of the water is from 5 vol% to 95 vol%: 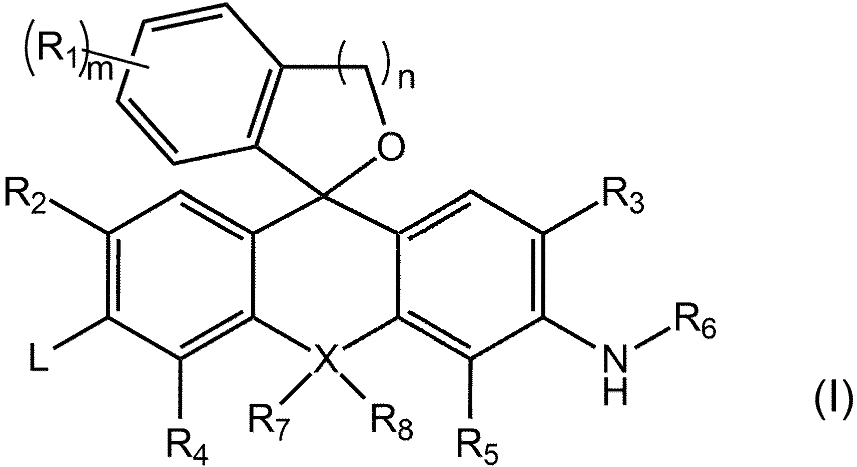
where:
R₁, if present, represents an identical or different monovalent substituent present on a benzene ring;
R₂ and R₃ each independently represent a hydrogen atom, an alkyl group having 1 to 6 carbon atoms, or a halogen atom;
R₄ and R₅ each independently represent a hydrogen atom, an alkyl group having 1 to 6 carbon atoms, or a halogen atom;
R₆ represents a hydrogen atom, an alkyl group having 1 to 5 carbon atoms, or a fluorinated alkyl group having 1 to 5 carbon atoms;
R₇ and R₈, if present, each independently represent an alkyl group having 1 to 6 carbon atoms or an aryl group;
X represents an oxygen atom, a silicon atom, or a carbon atom;
when X represents an oxygen atom, R₇ and R₈ are absent;
"n" represents an integer of from 1 to 3; and
L is selected from any groups of the following formulae (1) to (6).

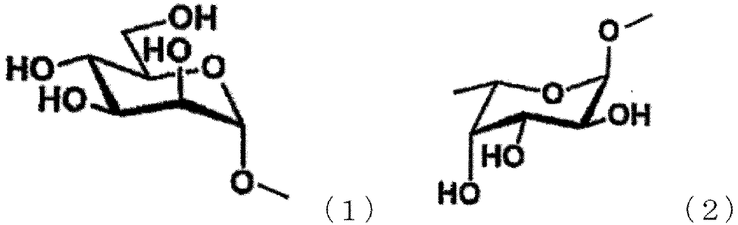

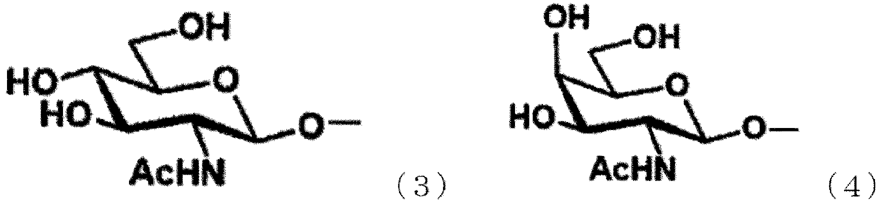

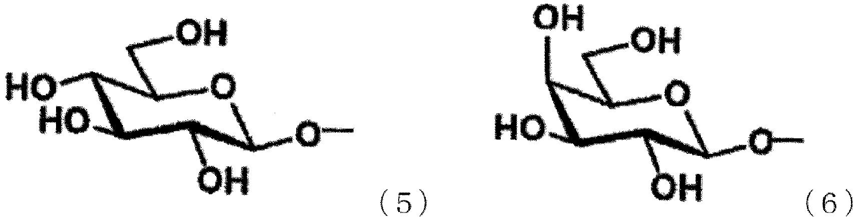

2. The fluorescent-probe solution according to claim 1, further comprising a solubilizer.

3. The fluorescent-probe solution according to claim 1 or 2, wherein the content of the organic solvent is from 40 vol% to 95 vol%.

4. The fluorescent-probe solution according to claim 1 or 2, wherein the organic solvent is dimethyl sulfoxide.

5. The fluorescent-probe solution according to claim 1 or 2, wherein the content of the water is from 20 vol% to 60 vol%.

6. The fluorescent-probe solution according to claim 1 or 2, wherein a content of the compound represented by the formula (I) is from 0.5 mM to 100 mM.

7. The fluorescent-probe solution according to claim 1 or 2, further comprising a buffer.

8. The fluorescent-probe solution according to claim 1 or 2, wherein the fluorescent-probe solution is stored in a vial having an opening sealed with a rubber stopper.
